# EUROPÄISCHE PATENTSCHRIFT

(11) **EP 0 514 627 B1**
(45) Veröffentlichungstag und Bekanntmachung des Hinweises auf die Patenterteilung: **31.01.1996**
(21) Anmeldenummer: 92103005.2
(22) Anmeldetag: 22.02.1992
(51) Int. Cl.: C07H 15/04

(54) **Verfahren zur Herstellung von Alkylpolyglycosiden**
Method for the preparation of alkylpolyglycosides
Procédé de préparation d'alcoylpolyglycosides

(30) Priorität: 22.05.1991 DE 4116665
(43) Veröffentlichungstag der Anmeldung: 25.11.1992
(73) Patentinhaber: HÜLS AKTIENGESELLSCHAFT, D-45764 Marl (DE)
(72) Erfinder: Kahsnitz, John, Dr., W-4358 Haltern (DE); Schmidt, Stefan, Dr., W-4358 Haltern (DE); Oberholz, Alfred, Dr., W-4370 Marl (DE)

(56) Entgegenhaltungen:
- EP-A- 0 301 298
- WO-A-90/06932
- DATABASE WPIL Section Ch, Week 9106, Derwent Publications Ltd., London, GB; Class A60, AN 91-040142

## Beschreibung

Die Erfindung betrifft ein verbessertes Verfahren zur Herstellung von Alkylpolyglycosiden durch Umsetzung von Alkylglycosiden mit Alkylgruppen mit 1 bis 6 C-Atomen mit Alkoholen mit 8 bis 18 C-Atomen.

Alkylpolyglycoside mit C₈- bis C₁₈-Alkylresten können ganz oder teilweise aus nachwachsenden Rohstoffen hergestellt werden. Diese Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside unter verminderter thermischer Belastung als transparente, farblich schöne wäßrige Lösungen hergestellt werden können.

Nach US 4 950 743 wird Glucosemonohydrat mit langkettigen Alkoholen direkt umgesetzt. Dabei muß zunächst das Hydratwasser durch Anlegen von Vakuum weitgehend entfernt werden, bevor man die Umsetzung durch Zusatz von Katalysator einleitet. Auch bei zweistufiger Verfahrensweise wird zunächst Glucosemonohydrat zusammen mit Butanol unter Vakuum fast quantitativ entwässert, bevor nach Katalysatorzugabe Butylglucosid hergestellt wird. Das Produkt wird anschließend im Rührkessel mit langkettigen Alkoholen umgesetzt. Nach beendeter Reaktion wird neutralisiert und vorzugsweise die Farbe durch Reduktion mit NaBH₄ verbessert.

Dieses Verfahren erfordert bei der direkten Umsetzung mit langkettigen Alkoholen einen Entwässerungsschritt und anschließend hohe Reaktionszeiten. Außerdem treten Phasentrennungsprobleme auf. Bei zweistufiger Fahrweise werden für die 2. Stufe noch 4,5 Stunden benötigt. Die Raum-Zeit-Ausbeuten sind hier gering.

In EP 0 377 831 wird bei der Umglycosidierung in einem Rührkessel eine saure Lösung von Butylglucosid in Butanol zu einem auf 100 bis 125 °C vorgeheizten, langkettigen Alkohol, der sauren Katalysator enthalten kann, zudosiert.

Hier wird das Produkt einer längeren thermischen Belastung ausgesetzt. Außerdem ist die Raum-Zeit-Ausbeute auch dieses Verfahrens noch nicht voll befriedigend.

Es bestand daher die Aufgabe, die Raum-Zeit-Ausbeute bei der Umglycosidierung zu verbessern und dabei die thermische Belastung von Produkt und Ausgangsverbindungen zu vermindern.

Diese Aufgabe wird erfindungsgemäß dadurch gelöst, daß man die Umsetzung in einer Reaktionskolonne durchführt.

Geeignete Reaktionskolonnen sind beispielsweise Glockenboden-, Siebboden-, Tunnelboden- oder Füllkörperkolonnen. Die Kolonnen besitzen vorzugsweise 2 bis 15 praktische Böden, wobei Kolonnen mit 2 bis 10 praktischen Böden ganz besonders bevorzugt eingesetzt werden. Die Kolonnen können in Gleich- oder Gegenstromfahrweise oder, in einer bevorzugten Ausführungsform, in einer modifizierten Gegenstromfahrweise betrieben werden.

Bei der Gleichstromfahrweise werden die Reaktionskomponenten in den oberen Teil der Kolonne eingeführt.

Unter dem oberen Teil der Kolonne wird dabei im Sinne dieser Erfindung das wirksame obere Viertel der Kolonne verstanden. Dazu gehören der Kolonnenkopf und ggf. einige der oberen Böden. Aufgesetzte Kolonnen oder Kolonnenteile zur Kondensation von Destillaten werden dabei nicht berücksichtigt.

Die Einsatzkomponenten sind das Alkylglycosid mit einer Alkylgruppe mit 1 bis 6 C-Atomen, der Alkohol mit 8 bis 18 C-Atomen und der Katalysator. Das hier verwendete Alkylglycosid kann beispielsweise in einem Rührkessel, einem Reaktionsrohr, einer Reaktionskolonne oder auch in einer Kombination von Reaktoren hergestellt werden.

In einer bevorzugten Ausführungsform werden mindestens 10 % des Alkohols mit 8 bis 18 C-Atomen zusammen mit den übrigen Einsatzkomponenten in den oberen Teil der Kolonne eingeleitet. Bis zu 90 % dieses langkettigen Alkohols werden dagegen in den unteren Teil der Kolonne eingeführt. Unter dem unteren Teil der Kolonne wird dabei die wirksame untere Hälfte der Kolonne verstanden.

In einer besonders bevorzugten Ausführungsform werden 20 bis 80 % des langkettigen Alkohols dampfförmig in den unteren Teil der Kolonne eingeleitet. In diesem Falle findet die Umglycosidierung auch unter Gegenstrombedingungen statt. Eine modifizierte Gegenstromfahrweise liegt dann vor, wenn dabei überschüssiger langkettiger Alkohol nicht am Kopf sondern am Boden der Kolonne herausgeführt wird.

Der Zuckeranteil der eingesetzten Alkylglycoside kann beispielsweise von Glucose, Mannose, Galactose oder Gulose hergeleitet werden. Als Alkylgruppen kommen zum Beispiel die Methyl-, Ethyl-, Butyl-, Pentyl- oder Hexylgruppe in Betracht. Die eingesetzten Alkylglycoside können auch in geringem Umfange oligomerisiert sein. So werden auch Produkte mit mittleren Oligomerisationsgraden von 1 bis 2 im Sinne dieser Erfindung als Alkylglycoside angesehen.

Die Alkylglycoside werden vorzugsweise zusammen mit einem Alkohol mit 1 bis 6 C-Atomen eingesetzt. Geeignete Alkohole sind beispielsweise Methanol, Ethanol, Butanol, Pentanol oder Hexanol. Im besonderen werden dabei, bezogen auf das Alkylglycosid, bis zu 5 Gewichtsteile Alkohol mit 1 bis 6 C-Atome mit eingesetzt.

In einer stark bevorzugten Ausführungsform wird Butylglucosid zusammen mit n-Butanol für die Umglycosidierung eingesetzt.

Geeignete langkettige Alkohole sind beispielsweise Octanol, Nonanol, Decanol, Dodecanol, Tetradecanol oder Hexadecanol.

Alkylglycosid und langkettiger Alkohol mit 8 bis 18 C-Atomen werden vorzugsweise im Molverhältnis von 1 : 2 bis 1 : 15 eingesetzt. Dabei werden Molverhältnisse von 1 : 4 bis 1 : 10 ganz besonders bevorzugt eingestellt.

Als Katalysatoren kommen vor allem Mineralsäuren wie Schwefel- oder Phosphorsäure sowie starke organische Säuren wie Benzolsulfonsäure, Cumolsulfonsäure oder p-Toluolsulfonsäure und auch saure Ionenaustauscher in Betracht. Wegen einer geringeren korrosiven Wirkung werden als Katalysatoren vorzugsweise organische Säuren eingesetzt.

Die Umglycosidierung wird meist bei Temperaturen von 100 bis 180 °C durchgeführt. Dabei werden Temperaturen von 120 bis 140 °C bevorzugt. Obwohl die Reaktion auch bei Normaldruck oder bei erhöhtem Druck ausgeführt werden kann, stellt man vorzugsweise einen verminderten Druck ein. Dabei werden Drücke von 10 bis 500 hPa ganz besonders bevorzugt.

Die nach dem vorliegenden Verfahren hergestellten Alkylpolyglycoside mit Alkylgruppen mit 8 bis 18 C-Atomen weisen im allgemeinen mittlere Polymerisationsgrade von 1,1 bis 5 auf. Der mittlere Polymerisationsgrad liegt vorzugsweise bei 1,2 bis 3.

Nach dem erfindungsgemäßen Verfahren wird die mittlere Verweilzeit der Reaktionsmischung auf wenige Minuten, beispielsweise auf 5 bis 15 Minuten, gesenkt. Gleichzeitig werden hohe Umsätze erzielt. Die Raum-Zeit-Ausbeute ist deshalb gegenüber der einer Umglycosidierung in einem Rührkessel erheblich verbessert.

Alkylpolyglycoside können nach dem erfindungsgemäßen Verfahren bei geringem apparativen Aufwand kontinuierlich hergestellt werden.

Außerdem werden die Produkte nach dem vorliegenden Verfahren thermisch wenig belastet. Deshalb kann man nach diesem Verfahren farblich verbesserte Alkylpolyglycoside erhalten.

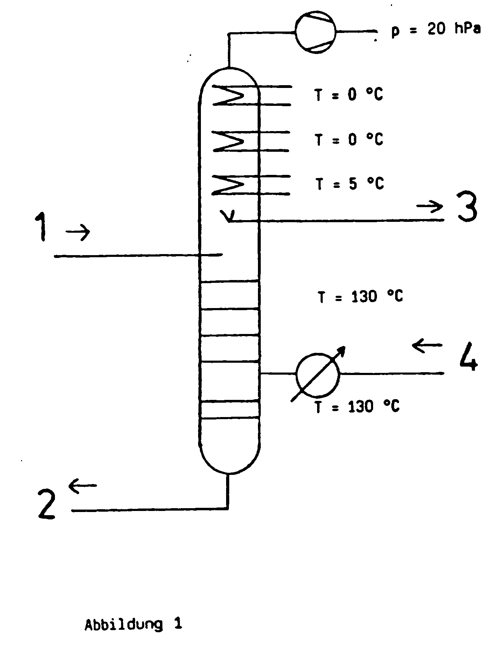

Bei der Methode der modifizierten Gegenstromfahrweise kann die Umglycosidierung allgemein gemäß Abbildung 1 wie folgt vorgenommen werden: Über Leitung 1 werden Alkylglycosid, kurzkettiger Alkohol, Katalysator und ein Teil des langkettigen Alkohols eingeleitet. Ein anderer Teil des langkettigen Alkohols wird als überhitzter Dampf über Leitung 4 in die Kolonne eingeleitet. Zusätzlich kann auch Inertgas über diese Leitung oder durch eine separate Leitung in die Blase auf die Kolonne gegeben werden. Der eingeführte kurzkettige Alkohol und der bei der Umglycosidierung gebildete kurzkettige Alkohol werden über Leitung 3 abgezogen. Überschüssiger langkettiger Alkohol und Alkylpolyglycosid werden gekühlt und über Leitung 2 ausgetragen.

Die Anlage zur Umglycosidierung kann eine Kolonne oder mehrere Kolonnen enthalten. Sie kann darüber hinaus auch Vor- und Nachreaktoren aufweisen, wobei jedoch die Hauptreaktion in den Kolonnen durchgeführt wird. Wegen der Vorreaktoren können die Kolonnen auch mit bereits teilweise umgesetzten Stoffgemischen betrieben werden.

Nach der Umglycosidierung wird das Reaktionsgemisch im allgemeinen neutralisiert. Die Neutralisation kann dabei nach bekannten Methoden mit basischen Stoffen, beispielsweise mit wäßriger Natronlauge, vorgenommen werden.

Die nachfolgende Destillation des Fettalkohols kann beispielsweise in einem Fallfilm- oder einem Dünnschichtverdampfer durchgeführt werden. Der abdestillierte Alkohol kann zurückgeführt und erneut für die Synthese verwendet werden. Den hier anfallenden Alkylpolyglycosiden können Fließmittel wie Glykole oder Glykolether in kleinen Mengen zugesetzt werden.

Anschließend werden die Alkylpolyglycoside gebleicht. Bei Anwendung von Ozon kann dabei die Bleichung auch in einer Blasensäule vorgenommen werden. Man kann die Produkte auch mit Aktivkohle behandeln oder ihnen Komplexierungsmittel zusetzen.

Die folgenden Beispiele sollen die Erfindung verdeutlichen.

### Beispiel 1

In einer Glockenbodenkolonne gemäß Abbildung 1 (6 Böden, statischer Hold-up = 200 ml/Boden, D = 80 mm, h = 100 mm/Boden) werden auf den 1. Boden (Leitung 1) 1,93 mol/h Butylglucosid, 10,21 mol/h Butanol, 12,70 mol/h n-Dodecanol sowie 0,060 mol/h p-Toluolsulfonsäure gegeben. Es werden keine weiteren Ströme in die Kolonne gefahren.
Die Kolonne wird bei einem Kopfdruck von 20 hPa (Absolutdruck) und den in Abbildung 1 angegebenen Temperaturen betrieben.
Am Kopf der Kolonne (Leitung 3) fällt Butanol und in der kalten Blase der Kolonne (Leitung 2) fettalkoholisches Alkylpolyglucosid an.

Das fettalkoholische Alkylpolyglycosid wird mit 5%iger methanolischer Kalilauge auf einen pH-Wert von 8 bis 9 eingestellt. Anschließend wird der Fettalkohol am Rotationsverdampfer abdestilliert. 50 g des erhaltenen Feststoffs werden in 50 ml Wasser gelöst. Durch Zusatz von 2 N Schwefelsäure wird ein pH-Wert von 7 eingestellt. Anschließend werden 5,3 ml einer 30%igen H₂O₂-Lösung zugesetzt. Es wird eine Stunde bei 80 °C gerührt. Dann werden 0,2 ml Natronwasserglas (Natriumsilikatlösung) zugesetzt, worauf der pH-Wert mit 2 N Natronlauge wieder auf 7 eingestellt wird. Nach Zugabe von 0,2 g Natriumhydrogencarbonat wird noch 30 Minuten nachgerührt. Die Iodfarbzahl (IFZ) der Lösung wird durch Vergleich mit einer Iodfarbskala nach DIN 6162 ermittelt.

| | |
|---|---|
| Umsatz des Butylglucosids: | > 68 % |
| Mittlere Verweilzeit: | 10 min |
| IFZ: | 7 -10 |

### Beispiel 2

In der Glockenbodenkolonne von Beispiel 1 werden auf den 1. Boden (Leitung 1) 1,96 mol/h Butylglucosid, 10,18 mol/h Butanol, 6,34 mol/h n-Dodecanol sowie 0,060 mol/h p-Toluolsulfonsäure gegeben. Außerdem werden auf den 5. Boden (Leitung 4) 6,44 mol/h n-Dodecanol als überhitzter Dampf in die Kolonne gefahren. Die Kolonne wird bei einem Kopfdruck von 20 hPa (Absolutdruck) und den in Abbildung 1 angegebenen Temperaturen betrieben.
Am Kopf der Kolonne (Leitung 3) fällt Butanol und in der kalten Blase der Kolonne (Leitung 2) fettalkoholisches Alkylpolyglucosid an. Die Aufarbeitung erfolgt wie in Beispiel 1.

| | |
|---|---|
| Umsatz des Butylglucosids: | > 96 % |
| Mittlere Verweilzeit: | 10 min |
| IFZ: | 7 -10 |

### Beispiel 3

In der Glockenbodenkolonne von Beispiel 1 werden auf den 1. Boden (Leitung 1) 1,92 mol/h Butylglucosid, 11,42 mol/h Butanol, 6,77 mol/h n-Dodecanol sowie 0,060 mol/h p-Toluolsulfonsäure gegeben. Auf den 5. Boden (Leitung 4) werden 6,43 mol/h n-Dodecanol als überhitzter Dampf in die Kolonne gefahren. Bis auf die Vorwärmertemperatur für den Fettalkoholstrom (Strom 4), die von 130 auf 160 °C erhöht ist, werden die in Abbildung 1 angegebenen Temperaturen beibehalten. Die Kolonne wird bei einem Kopfdruck von 18 hPa (Absolutdruck) betrieben.
Am Kopf der Kolonne fällt Butanol und in der kalten Blase der Kolonne fettalkoholisches Alkylpolyglucosid an.
Die Aufarbeitung erfolgt wie in Beispiel 1.

| | |
|---|---|
| Umsatz des Butylglucosids: | 99,2 % |
| Mittlere Verweilzeit: | 10 min |
| IFZ: | 7 -10 |

### Vergleichsbeispiel A

In eine 2stufige Rührkesselkaskade, die aus zwei 2,5-l-Rührreaktoren besteht, werden 2,05 l/h einer 35%igen Lösung von Butylglucosid in Butanol, die außerdem 0,65 % p-Toluolsulfonsäure enthält, und 2,5 l/h Fettalkohol, der aus einem Gemisch aus 70 % n-Dodecanol und 30 % n-Tetradecanol besteht, geleitet. Die Rührreaktoren werden im Vakuum auf Siedetemperatur erhitzt, so daß Butanol kontinuierlich abdestilliert. Das flüssige Reaktionsprodukt des 2. Reaktors ist eine etwa 25%ige Lösung von Alkylpolyglucosid in Fettalkohol.

| | |
|---|---|
| Umsatz des Butylglucosids: | > 99 % |
| Mittlere Verweilzeit: | 65 min |
| IFZ: | 20 |

## Patentansprüche

1. Verfahren zur Herstellung von Alkylpolyglycosiden aus Alkylglycosiden mit Alkylgruppen mit 1 bis 6 C-Atomen und Alkoholen mit 8 bis 18 C-Atomen,
dadurch gekennzeichnet,
daß man die Umsetzung in einer Reaktionskolonne durchführt.

2. Verfahren nach Anspruch 1,
dadurch gekennzeichnet,
daß man die Reaktionskomponenten in den oberen Teil der Kolonne einführt.

3. Verfahren nach Anspruch 1,
dadurch gekennzeichnet,
daß man mindestens 10 % des Alkohols mit 8 bis 18 C-Atomen in den oberen Teil und bis zu 90 % dieses Alkohols in den unteren Teil der Kolonne einleitet.

4. Verfahren nach Anspruch 3,
dadurch gekennzeichnet,
daß man 20 bis 80 % des Alkohols dampfförmig in den unteren Teil der Kolonne einspeist.

5. Verfahren nach Anspruch 1,
dadurch gekennzeichnet,
daß man das Alkylglycosid zusammen mit einem Alkohol mit 1 bis 6 C-Atomen einführt.

6. Verfahren nach Anspruch 5,
dadurch gekennzeichnet,
daß man pro Gewichtsteil Alkylglycosid bis zu 5 Gewichtsteile Alkohol mit 1 bis 6 C-Atomen einsetzt.

7. Verfahren nach Anspruch 5,
dadurch gekennzeichnet,
daß man Butylglucosid in Butanol verwendet.

8. Verfahren nach Anspruch 1,
dadurch gekennzeichnet,
daß man Alkylglycosid und Alkohol mit 8 bis 18 C-Atomen im Molverhältnis von 1 : 2 bis 1 : 15 einsetzt.

## Claims

1. A process for the preparation of alkyl polyglycosides from alkyl glycosides having alkyl groups having 1 to 6 C atoms, and alcohols having 8 to 18 C atoms, characterized in that the reaction is carried out in a reaction column.

2. A process according to claim 1, characterized in that the reaction components are introduced into the upper part of the column.

3. A process according to claim 1, characterized in that at least 10% of the alcohol having 8 to 18 C atoms is passed into the upper part and up to 90% of this alcohol is passed into the lower part of the column.

4. A process according to claim 3, characterized in that from 20 to 80% of the alcohol is fed into the lower part of the column as a vapour.

5. A process according to claim 1, characterized in that the alkyl glycoside is introduced together with an alcohol having 1 to 6 C atoms.

6. A process according to claim 5, characterized in that up to 5 parts by weight of alcohol having 1 to 6 C atoms are employed per part by weight of alkyl glycoside.

7. A process according to claim 5, characterized in that butyl glycoside in butanol is used.

8. A process according to claim 1, characterized in that alkyl glycoside and alcohol having 8 to 18 C atoms are employed in a molar ratio of from 1 : 2 to 1 : 15.

## Revendications

1. Procédé de préparation d'alkylpolyglycosides à partir d'alkylglycosides comportant des groupes alkyle ayant de 1 à 6 atomes de carbone et d'alcools ayant de 8 à 18 atomes de carbone, caractérisé en ce qu'on met en oeuvre la réaction dans une colonne de réaction.

2. Procédé selon la revendication 1, caractérisé en ce que les constituants de la réaction sont introduits dans la partie supérieure de la colonne.

3. Procédé selon la revendication 1, caractérisé en ce qu'on introduit au moins 10 % de l'alcool ayant de 8 à 18 atomes de carbone dans la partie supérieure de la colonne, et jusqu'à 90 % de cet alcool dans la partie inférieure de la colonne.

4. Procédé selon la revendication 3, caractérisé en ce qu'on introduit de 20 à 80 % de l'alcool, sous forme vapeur, dans la partie inférieure de la colonne.

5. Procédé selon la revendication 1, caractérisé en ce qu'on introduit l'alkylglycoside en même temps qu'un alcool ayant de 1 à 6 atomes de carbone.

6. Procédé selon la revendication 5, caractérisé en ce qu'on utilise par partie en poids d'alkylglycoside jusqu'à 5 parties en poids d'un alcool ayant de 1 à 6 atomes de carbone.

7. Procédé selon la revendication 5, caractérisé en ce qu'on utilise du butylglucoside dans du butanol.

8. Procédé selon la revendication 1, caractérisé en ce qu'on utilise l'alkylglucoside et l'alcool ayant de 8 à 18 atomes de carbone selon un rapport en moles de 1:2 à 1:15.
